# EUROPÄISCHE PATENTSCHRIFT

(11) **EP 2 840 954 B1**
(45) Veröffentlichungstag und Bekanntmachung des Hinweises auf die Patenterteilung: **30.03.2016**
(21) Anmeldenummer: 13718586.4
(22) Anmeldetag: 25.04.2013
(51) Int. Cl.: A61B 5/00, A61B 5/0215, A61M 1/10, A61M 25/01

(54) **KATHETERSYSTEM UND INTRAVASALE BLUTPUMPE MIT DIESEM KATHETERSYSTEM**
CATHETER SYSTEM AND INTRAVASCULAR BLOOD PUMP COMPRISING SAID CATHETER SYSTEM
SYSTÈME DE CATHÉTER ET POMPE À SANG INTRA-VASCULAIRE POURVUE DE CE SYSTÈME DE CATHÉTER

(30) Priorität: 27.04.2012 DE 102012207056
(43) Veröffentlichungstag der Anmeldung: 04.03.2015
(73) Patentinhaber: Abiomed Europe GmbH, 52074 Aachen (DE)
(72) Erfinder: SPANIER, Gerd, 52074 Aachen (DE); SIESS, Thorsten, 52074 Aachen (DE)
(74) Vertreter: Klunker . Schmitt-Nilson . Hirsch
(86) Internationale Anmeldenummer: PCT/EP2013/058636
(87) Internationale Veröffentlichungsnummer: WO 2013/160405

(56) Entgegenhaltungen:
- WO-A1-2011/039091
- US-A- 5 565 976
- US-A1- 2009 149 810
- US-A1- 2011 040 231

## Beschreibung

Die Erfindung betrifft allgemein ein System, umfassend einen Katheter, insbesondere einen Druckmesskatheter, und im speziellen eine intravasale Blutpumpe mit einem solchen Kathetersystem.

In WO 2011/039091 A1 wird im Zusammenhang mit einem Herzunterstützungssystem ein Druckmesskatheter beschrieben, der einen Katheterschlauch und einen Drucksensor zur Messung des Druckes distal von dem Katheterschlauch aufweist. Konkret weist der Druckmesskatheter einen optischen Drucksensor und ein langgestrecktes Rohr aus Metall oder aus einem hochfesten Kunststoff, zum Beispiel PEEK, auf, durch das eine lose verlegte Lichtleitfaser des optischen Drucksensors verläuft. Am vorderen (distalen) Ende des Druckmesskatheters befindet sich ein Sensorkopf, der nach dem Fabri-Perot-Prinzip arbeitet. Der Sensorkopf besitzt eine Kavität, die einerseits durch eine dünne, druckempfindliche Glasmembran abgeschlossen wird und in die andererseits das Ende der Lichtleitfaser hineinragt. Die druckempfindliche Glasmembran verformt sich in Abhängigkeit von der Größe des auf den Sensorkopf einwirkenden Drucks. Durch die Reflexion an der Glasmembran wird das aus der Lichtleitfaser austretende Licht modulierend reflektiert und wieder in die Lichtleitfaser eingespeist. Am proximalen Ende der Lichtleitfaser befindet sich eine Auswerteeinheit mit integrierter CCD-Kamera, die das erhaltene Licht in Form eines Interferenzmusters auswertet. In Abhängigkeit hiervon wird ein druckabhängiges elektrisches Signal erzeugt. Insgesamt handelt es sich somit um einen optoelektronischen Drucksensor.

Der Druckmesskatheter wird im Zusammenhang mit intravasalen Herzunterstützungssystemen, wie beispielsweise einer intraarteriellen Ballonpumpe (IABP) oder einer intravasalen Rotationsblutpumpe, verwendet, indem das betreffende Herzunterstützungssystem zunächst mittels eines Katheterschlauchs an die gewünschte Stelle des vaskulären Systems des Patienten vorgeschoben wird, also beispielsweise in die Aorta oder in eine Herzkammer. Der Druckmesskatheter einschließlich des die Lichtleitfaser umgebenden Rohres ist relativ zu diesem Katheterschlauch in dessen Längsrichtung verschiebbar und wird nachträglich in das Lumen des Katheterschlauchs eingeführt, durch den Katheterschlauch vorgeschoben und tritt aus dessen Ende aus. Wenn der Sensorkopf die vorgesehene Messstelle erreicht hat, wird das Rohr des Druckmesskatheters zurückgezogen, kann aber auch als ein fester Bestandteil des Druckmesskatheters an Ort und Stelle verbleiben. Im Zusammenhang mit einer Rotationsblutpumpe wird vorgeschlagen, den Druckmesskatheter weit über das distale Ende des Katheterschlauchs hinaus an der Pumpeneinrichtung der Rotationsblutpumpe vorbei zu schieben, so dass er die Aortenklappe passiert und mit seinem Sensorkopf in den linken Ventrikel hineinragt, um so den Ventrikeldruck zu messen.

Problematisch bei der Navigation mittels Kathetern innerhalb des vaskulären Systems sind zu passierende Krümmungen, Gefäßverzweigungen oder Hindernisse, wie zum Beispiel Herzklappen. An solchen Stellen kann sich der Katheter verhaken und aufgrund seiner Flexibilität umbiegen, anstatt sich weiter voranzuschieben. Man spricht vom "Knicken". Ein solches Knicken ist für den behandelnden Arzt nicht immer spürbar. Da die vom Drucksensor gelieferten physiologischen Drucksignale häufig nicht eindeutig sind, kann es daher vorkommen, dass der Arzt die Fehlplatzierung des Katheters nicht erkennt, sondern der irrigen Annahme ist, der Katheter habe die gewünschte Platzierung erreicht.

Aufgabe der Erfindung ist es daher zu vermeiden, dass ein Katheter irrtümlich im geknickten Zustand in ein Gefäß des Patienten, sei es ein Blutgefäß oder ein anderes Gefäß, verlegt wird.

Diese Aufgabe wird durch die Merkmale des unabhängigen Anspruchs gelöst. In davon abhängigen Ansprüchen sind vorteilhafte Weiterbildungen und Ausgestaltungen der Erfindung angegeben.

Gemäß einer bevorzugten Ausführungsform der Erfindung wird der Katheter mit einem Knicksensor ausgestattet. Sobald der Katheter knickt, wird dies detektiert und in einer Auswerteeinrichtung ausgewertet, die beispielsweise ein entsprechendes Signal für den behandelnden Arzt erzeugen kann. Der Knicksensor kann absolut arbeiten, d. h. wenn ein vorgegebenes Knickereignis detektiert wird, das beispielsweise ab einem definierten Biegeradius des Katheters eintritt, so wird ein Alarm ausgelöst. Er kann aber auch relativ arbeiten und den Grad des Knickens anzeigen, wobei ggf. bei Erreichen eines Grenzwertes ein Alarm ausgelöst wird.

Der Knicksensor erstreckt sich vorzugsweise über die gesamte Länge des Katheters und kann ein Knicken des Katheters über dessen gesamte Länge detektieren. Auf diese Weise werden auch solche Knickereignisse detektiert, die beispielsweise auftreten, wenn sich das distale Ende des Katheters verhakt, der Katheter aber weit entfernt von der Katheterspitze an einer solchen Stelle des vaskulären Systems knickt, an der dafür genug Raum vorhanden ist, beispielsweise an einer größeren Gefäßverzweigung.

Gemäß einem besonders bevorzugten Ausführungsbeispiel umfasst der Knicksensor mindestens eine Lichtleitfaser. Lichtleitfasern leiten Licht quasi verlustfrei auch im gebogenen Zustand. Ab einem vorbestimmten Biegeradius koppelt allerdings ein Teil des Lichts seitlich aus der Lichtleitfaser aus. Welches der kritische Biegeradius ist, hängt von dem Material der Lichtleitfaser, hier speziell von den Brechungsindizes der Glasschichten, und dem an die Oberfläche der Lichtleitfaser angrenzenden Material ab. Insbesondere hängt dies von dem Aufbau der Glasfaser ab, speziell der Ausführung der radial symmetrischen Reflexionsschicht in der Glasfaser, die gebildet wird durch die Grenzfläche zwischen dem Lichtleitfaserkern (Core) und dem Lichtleitfasermantel (Cladding). Hierbei kann es sich sowohl um eine graduelle Ausführung der Glasfaser (Gradientenindexfaser) als auch um einen direkten Übergang (Stufenindexfaser) handeln. Glasfasern sind darüberhinaus außen zu ihrem mechanischen Schutz in der Regel kunststoffbeschichtet, etwa mit Polyimid.

Der besondere Vorteil der Verwendung einer Lichtleitfaser als Knicksensor besteht darin, dass eine Lichtleitfaser einfach aufgebaut und dementsprechend billig herzustellen ist und dass damit insbesondere ein Knicken über den gesamten Längenbereich des Katheters möglich ist, in dem die Lichtleitfaser verlegt ist. Umgekehrt kann auch ein spezieller Teil des Katheters, wie die Katheterspitze, besonders knickempfindlich angelegt werden, so dass bevorzugt dort das Knicken detektiert wird.

Durch eine geeignete Wahl der Materialpaarung zwischen dem das Licht leitenden Lichtleitfaserkern und dem den Lichtleitfaserkern umgebenen Material kann in gewissen Grenzen die Sensitivität des Knicksensors voreingestellt werden, so dass ab einem vorgegebenen Biegeradius Licht tatsächlich aus der Lichtleitfaser auskoppelt. Auf diese Weise oder durch Einsatz mehrerer Fasern unterschiedlicher Länge oder mit Licht unterschiedlich leitenden Segmenten ist sogar eine ortsabhängige Detektion der Biegung möglich.

Die Lichtleitfaser wird dementsprechend an eine Auswerteeinrichtung angeschlossen, die dazu eingerichtet ist, eine durch die Lichtleitfaser geleitete vorgegebene Lichtmenge dahingehend auszuwerten, ob ein Teil der Lichtmenge entlang der Länge der Lichtleitfaser aus der Lichtleitfaser ausgekoppelt wird. Dazu wird vorzugsweise Licht ins proximale Ende der Lichtleitfaser eingekoppelt, am distalen Ende der Lichtleitfaser reflektiert und die am proximalen Ende eintreffende Menge des reflektierten Lichts gemessen. Verändert sich diese Lichtmenge und infolgedessen das Signal-zu-Rauschverhältnis, so ist daraus zu schließen, dass ein Teil der Lichtmenge über die Länge der Lichtleitfaser ausgekoppelt wurde. Sobald dieser ausgekoppelte Teil der Lichtmenge einen vorgegebenen Grenzwert erreicht oder überschreitet, der auch sehr gering angesetzt werden kann, kann auf ein Knicken des Katheters zurückgeschlossen werden. Die Auswerteeinrichtung erzeugt dann vorzugsweise ein Alarmsignal in optischer, akustischer oder sonstiger Weise. Bei entsprechender Auslegung des Katheters kann der Lichtleiter derart geknickt werden, dass dies reversibel ohne Bruch erfolgt, so dass der Anwender durch geeignete Manipulation in die Lage versetzt wird, starke Biegungen/Knicken zu revidieren.

Die Verwendung einer Lichtleitfaser als Knicksensor ist dementsprechend sehr zuverlässig. Von Bedeutung ist allerdings, dass die Lichtleitfaser beim Eintritt eines Knickereignisses selbst nicht bricht. Im Falle von Kunststofffasern können Weissbrüche auftreten, und im Falle von Glasfasern können die Fasern insgesamt brechen. Zwar würde auch dies im Ergebnis von der Auswerteeinrichtung richtigerweise als Knickereignis identifiziert werden. Jedoch wäre der Knicksensor dann zerstört und nicht mehr brauchbar. Um auch nach extremen Knickereignissen weiter funktionsfähig zu sein, ist es daher vorteilhaft, Lichtleitfasern aus Kunststoff mit einem Durchmesser von 250 µm oder weniger auszubilden. Umfasst die Lichtleitfaser eine Glasfaser, so sollte der Durchmesser der Lichtleitfaser nicht mehr als 150 µm betragen. Die angegebenen Durchmesserwerte betreffen jeweils den Gesamtdurchmesser der Lichtleitfaser einschließlich etwaiger Beschichtungen des das Licht leitenden Lichtleitfaserkerns. Bruch- und auch Weissbrüche der Fasern können ebenfalls detektiert werden. Dadurch ist es möglich, dem Anwender den Ausfall der Messeinrichtung mitzuteilen und somit zu vermeiden, dass mit einem defekten System weitere Platzierungsversuche unternommen werden. Dies ist für den Einsatz als medizintechnischer Sensor wichtig.

Die Lichtleitfaser ist vorzugsweise in dem Katheter frei beweglich verlegt. Sie kann aber zum Schutz gegen andere in dem Katheter verlegte Einrichtungen in einem separaten Lumen frei beweglich verlegt sein. Das Lumen, in dem die Lichtleitfaser verläuft, besteht vorzugsweise aus einem Material, das eine Formgedächtnislegierung umfasst, insbesondere aus sogenanntem "Nitinol". Formgedächtnislegierungen (englisch: shape memory alloy, SMA) werden oft auch als Memorymetalle bezeichnet, weil sie sich an eine frühere Formgebung trotz nachfolgender starker Verformung scheinbar "erinnern" können. Dieser Erinnerungsvorgang ist temperaturabhängig. Der besondere Nutzen einer Formgedächtnislegierung als Material für das Lumen der Lichtleitfaser liegt allerdings nicht in der temperaturabhängigen Erinnerungsfähigkeit des Materials, sondern darin, dass Formgedächtnislegierungen ein superelastisches Verhalten zeigen. So bewegt sich auch das Lumen selbst, in dem die Lichtleitfaser aufgenommen ist, nach einem Knickereignis wieder elastisch in seine Ausgangsform zurück.

Der Außendurchmesser eines solchen Lumens aus einer Formgedächtnislegierung beträgt vorzugsweise 330 µm oder weniger, besonders bevorzugt 220 µm oder weniger, und der Innendurchmesser beträgt vorzugsweise 230 µm oder weniger bzw. besonders bevorzugt 150 µm oder weniger. Nitinol-Röhrchen, also Röhrchen aus einer entsprechenden Nickel-Titan-Legierung mit Formgedächtniseigenschaften, sind mit den vorgenannten Durchmesserabmessungen kommerziell verfügbar.

Der Katheter kann alternativ auch aus einem Polymer und hier bevorzugt Polyurethan gefertigt sein, da auch dieses Material kurzzeitig über ein Formgedächtnis verfügt und sich weitgehend reversibel "entknicken" kann. Das Polymermaterial ist innenseitig vorzugsweise gleitbeschichtet und die Lichtleitfaser darin frei verlegt.

Die Erfindung ist besonders vorteilhaft integrierbar in einen optischen Drucksensor, der bereits selbst eine Lichtleitfaser umfasst, wie zum Beispiel der eingangs beschriebene optische Drucksensor, der nach dem Fabri-Perot-Prinzip arbeitet. Da die Lichtleitfaser im optischen Drucksensor bereits vorhanden und das durch die Lichtleitfaser geleitete Licht ohnehin von einer Auswerteeinrichtung ausgewertet wird, braucht die Auswerteeinrichtung lediglich dahingehend angepasst zu werden, dass überprüft wird, ob ein Teil der durch die Lichtleitfaser geleiteten Lichtmenge aus der Lichtleitfaser ausgekoppelt wird.

Ein entsprechender optischer Drucksensor kann als selbstständiger Katheter ausgebildet sein, der entweder isoliert ins vaskuläre System des Patienten eingeführt wird oder Teil eines größeren Katheters ist. Besonders vorteilhaft ist es, wenn der Knicksensor bzw. der als Knicksensor fungierende optische Drucksensor Teil eines Katheters einer intravasalen Blutpumpe ist, mit dem die Blutpumpe innerhalb des vaskulären Systems eines Patienten navigiert wird. In diesem Fall wird der Knicksensor, d. h. die Lichtleitfaser oder das Lumen, in dem die Lichtleitfaser verlegt ist, fest mit der Blutpumpe verbunden. Dazu wird der Knicksensor entlang des Katheters verlegt, entweder innerhalb und/ oder außerhalb, und zumindest der Sensorkopf, also das distale Ende des Knicksensors, wird fest mit der Blutpumpe verbunden, um seine Knicksensorfunktion zuverlässig erfüllen zu können.

Dabei kann sich der Knicksensor auch über den Katheter hinaus entlang der Blutpumpe zum Beispiel bis ans distale Ende der Blutpumpe erstrecken. Dies ist etwa in den Fällen sinnvoll, in denen die Blutpumpe an ihrem distalen Ende eine flexible Strömungskanüle besitzt, die selbst knickgefährdet ist. Diese Ausführungsform ist besonders vorteilhaft, da beispielsweise die unmittelbare Klappenlage durch Biegen der Kanüle oder des distalen Endes der Kanüle detektiert werden und so eine Passage der Herzklappe retrograd ohne zusätzliche bildgebende Verfahren erfolgen kann. Im Detail bedeutet dies, dass der Sensor nicht nur den Übergang vom Druck vor der Klappe zum Druck hinter der Klappe dokumentiert sondern durch Deformation zusätzlich genau ortsabhängig die Klappenpassage unterstützt. Durch Kontrolle des Biegeradius der Kanüle kann die Kraft, mit der gegen die Klappe gedrückt wird, indirekt gemessen werden und so eine atraumatische Passage ermöglicht werden.

Nachfolgend wir die Erfindung anhand der begleitenden Zeichnungen beispielhaft beschrieben. Darin zeigen:
Figur 1 eine durch die Aorta verlegte Blutpumpe, die sich durch die Aortenklappe bis in den linken Ventrikel erstreckt, mit integriertem Druck- und Knicksensor, und
Figur 2 einen optischen Drucksensor mit Lichtleitfaser.

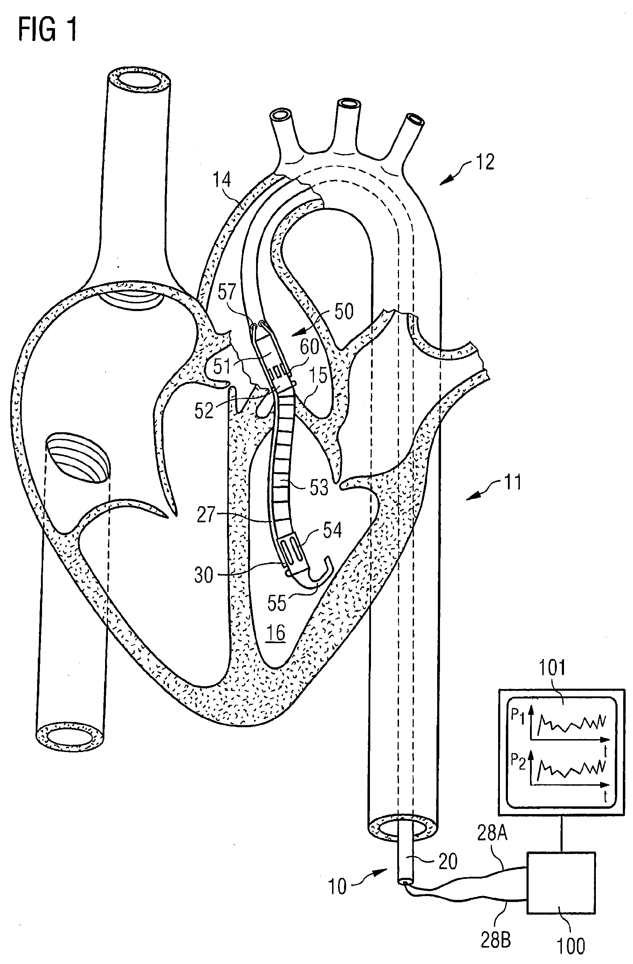

Figur 1 zeigt eine intravasale Blutpumpe mit einem Katheter 10, der retrograd in die Aorta descendens 11 eingeführt ist. Die Aorta descendens ist Bestandteil der Aorta 12, die vom Herzen zunächst aufsteigt und dann abwärts führt und den Aortenbogen 14 aufweist. Am Beginn der Aorta 12 befindet sich die Aortenklappe 15, die den linken Ventrikel 16 mit der Aorta 12 verbindet und durch die hindurch sich die intravasale Blutpumpe erstreckt. Die intravasale Blutpumpe umfasst zusätzlich zu dem Katheter 10 eine am distalen Ende des Katheterschlauchs 20 befestigte rotatorische Pumpeinrichtung 50, die einen Motorteil 51 und einen in axialem Abstand hiervon angeordneten Pumpenteil 52 sowie eine von dem Ansaugende des Pumpenteils 52 in distaler Richtung abstehende Strömungskanüle 53 aufweist, an deren Ende sich ein Saugeinlass 54 befindet. Distal von dem Saugeinlass 54 ist eine weichflexible Spitze 55 vorgesehen, die beispielsweise als "Pigtail" oder in J-Form ausgeführt sein kann. Durch den Katheterschlauch 20 verlaufen verschiedene Leitungen und Einrichtungen, die für den Betrieb der Pumpeinrichtung 50 von Bedeutung sind. Davon sind in Figur 1 lediglich zwei Lichtleitfasern 28A, 28B gezeigt, die mit ihrem proximalen Ende an eine Auswerteeinrichtung 100 angeschlossen sind. Diese Lichtleitfasern 28A, 28B sind jeweils Teil eines optischen Drucksensors, deren Sensorköpfe 30 und 60 sich einerseits außen am Gehäuse des Pumpenteils 52 und andererseits außen am Saugeinlass 54 befinden. Der von den Sensorköpfen 30 und 60 übermittelte Druck wird in der Auswerteeinrichtung 100 in elektrische Signale umgewandelt und z.B. auf einem Display 101 angezeigt.

Durch die Messung sowohl des Aortendrucks mittels des Sensorkopfs 60 als auch des Ventrikeldrucks mittels des Sensorkopfs 30 sind zusätzlich zum eigentlichen Drucksignal z.B. eine Kontraktibilitätsmessung, bei der die Erholung des Herzens gemessen wird, sowie die Ermittlung der Druckdifferenz, die zur Flussberechnung der Pumpeinrichtung 50 hinzugezogen wird, möglich.

Der distale Sensorkopf 30 kann zudem bis in die weichflexible Spitze 55 hineinreichen, wodurch speziell bei der retrograden Klappenpassage der Druckübergang vom Aortendruck auf den Ventrikeldruck an der Spitze 55 der Pumpe erfasst wird. Zudem kann so das Biegen der Spitze 55 sehr sensitiv detektiert werden, was eine vereinfachte Klappenpassage ermöglicht. Bei wandnaher Lage der Pumpe, wie in Fig. 1, kann zudem ein zu großer Druck auf die Herzwand infolge des Biegens oder Knickens detektiert werden. Letzteres kann auch zum Ansaugen des Einlasses an kardialen Strukturen führen. Detektion dieses Zustands kann vom Anwender durch Drehen oder Zurückziehen der Pumpe revidiert werden.

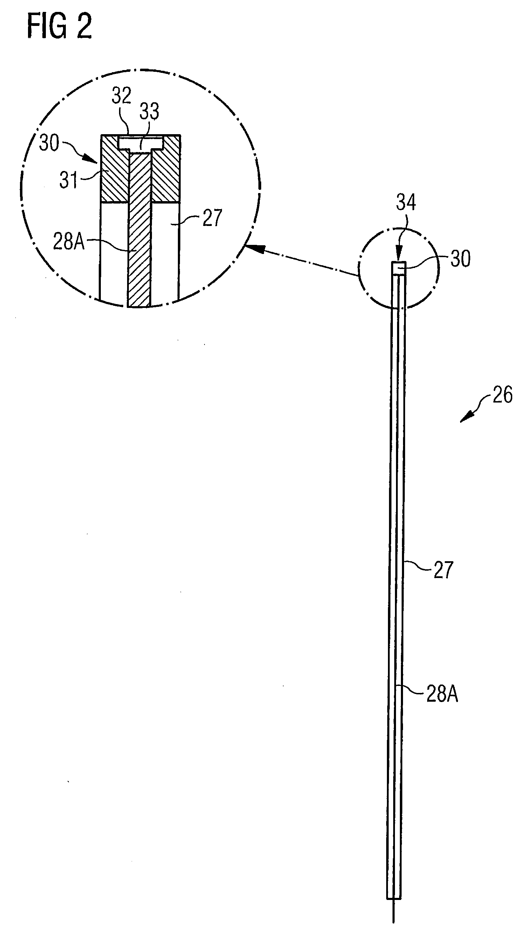

Das Prinzip der elektrooptischen Druckmessung wird nachfolgend anhand der Figur 2 näher erläutert. Figur 2 zeigt einen Druckmesskatheter 26 mit einem Lumen 27, in dem eine Lichtleitfaser 28A (es könnten auch mehrere Lichtleitfasern sein) frei beweglich ist. Das Lumen 27 kann vorzugsweise aus Nitinol oder einer anderen Formgedächtnislegierung oder einem Polymerschlauch bestehen, an einer Austrittsstelle 57 aus dem Katheterschlauch 20 austreten und an der flexiblen Strömungskanüle 53 außen entlang geführt sein. Innerhalb des Katheterschlauchs 20 kann auf das separate Lumen 27 verzichtet werden. Am distalen Ende 34 der Lichtleitfaser 28A weist der Druckmesskatheter einen Sensorkopf 30 mit einem Kopfgehäuse 31 auf, das eine dünne Glasmembran 32 enthält, die eine Kavität 33 abschließt. Die Lichtleitfaser 28A braucht hier nicht zwangsläufig die Kavität abzuschließen. Lediglich auf ein verlustarmes Ein- und Auskoppeln des Lichts aus und in die Faser 28A muss geachtet werden. Die Glasmembran 32 ist druckempfindlich und verformt sich in Abhängigkeit von der Größe eines auf den Sensorkopf 30 einwirkenden Drucks. Durch die Reflexion an der Membran wird das aus der Lichtleitfaser 28A austretende Licht modulierend reflektiert und wieder in die Lichtleitfaser eingekoppelt. Am proximalen Ende der Lichtleitfaser 28A, d. h. in der Auswerteeinrichtung 100, befindet sich eine Digitalkamera, z.B. eine CCD-Kamera oder ein CMOS, die das eintreffende Licht in Form eines Interferenzmusters auswertet. In Abhängigkeit davon wird ein druckabhängiges elektrisches Signal erzeugt. Die Auswertung des von der Kamera gelieferten optischen Bildes bzw. optischen Musters und die Berechnung des Drucks erfolgen durch einen an die Kamera angeschlossenen Rechner, der auch die Stromzufuhr zu der motorisch betriebenen Pumpeinrichtung 50 in Abhängigkeit von der erfolgten Auswertung des Drucksignals steuert.

Erfindungsgemäß dient zumindest die Lichtleitfaser 28A auch als Knicksensor und erfüllt somit eine zweite Funktionalität. Es ist aber auch möglich, eine separate Lichtleitfaser im Katheterschlauch 20 und ggf. über den Katheterschlauch hinaus als Knicksensor vorzusehen. In diesem Falle braucht der Sensorkopf 30 nicht so kompliziert aufgebaut zu sein, wie in Bezug auf Figur 2 erläutert, sondern es genügt, wenn die distale Endfläche 33 der Lichtleitfaser 28A das in der Lichtleitfaser 28A geleitete Licht zurückspiegelt. Dieselbe Auswerteeinrichtung 100 kann verwendet werden und muss lediglich dazu eingerichtet werden zu messen, in welchem Maße die in die Lichtleitfaser 28A eingekoppelte Lichtmenge zurückkommt. Dazu kann die Menge des pro Zeiteinheit von der CCD-Kamera oder einer anderen lichtsensitiven Einrichtung erfassten Lichts aufsummiert und etwaige Schwankungen erfasst und als Knickereignis angezeigt werden. Abhängig von der Menge der Lichtauskopplung kann auch auf einen Biegeradius geschlossen werden, noch bevor ein tatsächliches Knickereignis auftritt.

## Patentansprüche

1. System, umfassend einen Katheter (10; 26) mit einem Knicksensor (28A, 100), wobei das System Teil einer intravasalen Blutpumpe ist, der Knicksensor mindestens eine Lichtleitfaser (28A) umfasst, die Lichtleitfaser (28A) Teil eines optischen Drucksensors (30-34) ist, der Knicksensor einen fest mit der Blutpumpe verbundenen Sensorkopf (30) besitzt, und wobei die Lichtleitfaser (28A) des Katheters (10; 26) an eine Auswerteeinrichtung (100) angeschlossen ist, welche dazu eingerichtet ist, eine durch die Lichtleitfaser (28A) geleitete vorgegebene Lichtmenge dahingehend auszuwerten, ob ein Teil der Lichtmenge entlang der Länge der Lichtleitfaser aus der Lichtleitfaser ausgekoppelt wird.

2. System nach Anspruch 1, wobei sich der Knicksensor über die gesamte Länge des Katheters (10; 26) erstreckt und eingerichtet ist, ein Knicken des Katheters über die gesamte Länge des Katheters zu detektieren.

3. System nach einem der Ansprüche 1 bis 2, wobei die Lichtleitfaser Segmente mit unterschiedlichen Lichtleiteigenschaften aufweist.

4. System nach einem der Ansprüche 1 bis 2, wobei der Knicksensor mehrere Lichtleitfasern unterschiedlicher Länge aufweist.

5. System nach einem der Ansprüche 1 bis 4, wobei die Lichtleitfaser (28A) eine Glasfaser umfasst und einen Durchmesser von 120 µm oder weniger besitzt.

6. System nach einem der Ansprüche 1 bis 4, wobei die Lichtleitfaser (28A) eine Kunststofffaser umfasst und einen Durchmesser von 250 µm oder weniger besitzt.

7. System nach einem der Ansprüche 1 bis 6, wobei die Lichtleitfaser (28A) in einem Lumen (20; 27) frei beweglich verlegt ist, welches aus einem Material besteht, das eine Formgedächtnislegierung umfasst.

8. System nach einem der Ansprüche 1 bis 6, wobei die Lichtleitfaser (28A) in einem Lumen (20; 27) frei beweglich verlegt ist, welches aus einem Polymermaterial besteht, das innen gleitbeschichtet ist.

9. System nach einem der Ansprüche 1 bis 8, wobei der Drucksensor (30-34) einen Sensorkopf (30) aufweist, der an einer distalen, weichflexiblen Spitze (55) des Katheters (10) angeordnet ist.

10. System nach einem der Ansprüche 1 bis 9, wobei die Auswerteeinrichtung (100) dazu eingerichtet ist, ein Alarmsignal zu erzeugen, wenn der ausgekoppelte Teil der Lichtmenge einen vorgegebenen Grenzwert erreicht oder überschreitet.

## Claims

1. A system comprising a catheter (10; 26) with a bend sensor (28A, 100), wherein the system forms part of an intravascular blood pump, the bend sensor comprises at least one fiber optical waveguide (28A), the fiber optical waveguide (28A) forms part of an optical pressure sensor (30-34), the bend sensor has a sensor head (30) permanently connected to the blood pump, and wherein the fiber optical waveguide (28A) of the catheter (10; 26) is connected to an evaluation device (100) adapted to evaluate the predetermined amount of light guided through the fiber optical waveguide (28A) for whether a portion of the amount of light is coupled out of the fiber optical waveguide along the length of the fiber optical waveguide.

2. The system according to claim 1, wherein the bend sensor extends over the complete length of the catheter (10; 26) and is adapted to detect a bending of the catheter over the complete length of the catheter.

3. The system according to any of the claims 1 to 2, wherein the fiber optical waveguide has segments with different light-guiding properties.

4. The system according to any of the claims 1 to 2, wherein the bend sensor has several fiber optical waveguides of different length.

5. The system according to any of the claims 1 to 4, wherein the fiber optical waveguide (28A) comprises a glass fiber and has a diameter of 120 µm or less.

6. The system according to any of the claims 1 to 4, wherein the fiber optical waveguide (28A) comprises a plastic fiber and has a diameter of 250 µm or less.

7. The system according to any of the claims 1 to 6, wherein the fiber optical waveguide (28A) is laid in freely movable fashion in a lumen (20; 27) consisting of a material comprising a shape memory alloy.

8. The system according to any of the claims 1 to 6, wherein the fiber optical waveguide (28A) is laid in freely movable fashion in a lumen (20; 27) consisting of a polymer material that is slide-coated on the inside.

9. The system according to any of the claims 1 to 8, wherein the pressure sensor (30-34) has a sensor head (30) that is arranged at a distal, soft-flexible tip (55) of the catheter (10).

10. The system according to any of the claims 1 to 9, wherein the evaluation device (100) is adapted to produce an alarm signal when the coupled-out portion of the amount of light reaches or overshoots a predetermined limit value.

## Revendications

1. Système comprenant un cathéter (10; 26) muni d'un capteur de flexion (28A, 100), le système faisant partie d'une pompe à sang intravasculaire, le capteur de flexion comprenant au moins une fibre optique (28A), la fibre optique (28A) faisant partie d'un capteur de pression optique (30-34), le capteur de flexion possédant une tête de capteur (30) fermement reliée à la pompe à sang, et la fibre optique (28A) du cathéter (10; 26) étant raccordée à un dispositif d'évaluation (100) qui est configuré pour évaluer une quantité prédéterminée de lumière conduite par la fibre optique (28A) de sorte à déceler si une partie de la quantité de lumière est découplée de la fibre optique le long de la longueur de la fibre optique.

2. Système selon la revendication 1, le capteur de flexion s'étendant sur toute la longueur du cathéter (10; 26) et étant configuré pour détecter sur toute la longueur de la fibre optique une flexion du cathéter.

3. Système selon une des revendications de 1 à 2, la fibre optique comportant des segments ayant différentes propriétés de transmission lumineuse.

4. Système selon une des revendications de 1 à 2, le capteur de flexion comportant plusieurs fibres optiques de différente longueur.

5. Système selon une des revendications de 1 à 4, la fibre optique (28A) comprenant une fibre de verre et ayant un diamètre de 120 µm ou moins.

6. Système selon une des revendications de 1 à 4, la fibre optique (28A) comprenant une fibre en matière plastique et ayant un diamètre de 250 µm ou moins.

7. Système selon une des revendications de 1 à 6, la fibre optique (28A) étant posée de façon librement mobile dans un lumen (20; 27) qui consiste en un matériau comprenant un alliage à mémoire de forme.

8. Système selon une des revendications de 1 à 6, la fibre optique (28A) étant posée de façon librement mobile dans un lumen (20; 27) qui consiste en un matériau polymère doté d'un revêtement de glissement à l'intérieur.

9. Système selon une des revendications de 1 à 8, le capteur de pression (30-44) comportant une tête de capteur (30) qui est agencée à une pointe (55) distale souple et flexible du cathéter (10).

10. Système selon une des revendications de 1 à 9, le dispositif d'évaluation (100) étant configuré pour générer un signal d'alarme quand la partie de la quantité de lumière découplée atteint ou dépasse une valeur limite prédéterminée.
